Europäisches Patentamt

European Patent Office

Office européen des brevets

(11) Publication number: **0 153 132**
**A1**

(12) **EUROPEAN PATENT APPLICATION**

(21) Application number: **85300917.3**

(22) Date of filing: **12.02.85**

(51) Int. Cl.⁴: **A 61 M 15/08**

---

(30) Priority: **13.02.84 AU 3586/84**

(43) Date of publication of application: **28.08.85**
**Bulletin 85/35**

(84) Designated Contracting States: **AT BE CH DE FR GB IT
LI LU NL SE**

(71) Applicant: **Forrest, Victoria Anne, 67/25A Marks Street,
Narrenburn New South Wales 2065 (AU)**

(72) Inventor: **Forrest, Victoria Anne, 67/25A Marks Street,
Narrenburn New South Wales 2065 (AU)**

(74) Representative: **Hartley, David et al, c/o Withers &
Rogers 4 Dyer's Buildings Holborn, London, EC1N 2JT
(GB)**

---

(54) **Surgical device for maintaining airways in the nasal passages.**

㉗ The specification discloses a unitary structure for use in nasal surgery comprising a curved plastics tube (1) having a cut-out portion (2) so that on being folded a pair of open ended joined tubes for insertion into the nasal passages is formed whereby blockage of the passage is minimised and oxygen depletion minimised.

## SURGICAL DEVICE FOR MAINTAINING

## AIRWAYS IN THE NASAL PASSSAGES

This invention relates to a surgical device for maintaining airways in the nasal passages of a human being, or an animal.

The present invention evolved as direct result of observations and experiences of the inventor in her capacity as registered general nurse and theatre sister at St. Vincent's Hospital, Sydney, Australia. It was noted that patients undergoing nasal surgery e.g. Septoplasty, in the post anaesthetic period and for twenty four hours afterwards exhibit extreme restlessness and great difficulty breathing through the nose. During this time, the patients are at risk. Should a complete or partial blockage of the nasal passages occur, the result is a depletion of oxygen intake and an increase in carbon dioxide retention. The risk of cardiac arrest can become acute.

Current post-operative procedures adopted by the surgeon may contribute to the rick of blockage. The most favoured procedure is to pack the nostrils with gauze impregnated with petroleum jelly. Other methods include the use of commercially available petroleum jelly gauze wicks. Some surgeons pack the nostrils with glove fingers improvised by cutting the fingers from a surgical glove, cutting of the finger tops and filling with petroleum jelly. Yet another means which is somewhat complex and expensive, is to fit in the nasal passages, a unitary plastic splint consisting of two limbs connected by a narrow strip. The splint has a separate breathing tube for each nasal passage.

One object of the present invention is to avoid the difficulties associated with conventional procedures and techniques.

According to the present invention I propose a device for maintaining airways in nasal passages comprising a length of tubular material having a cut-out portion preferably at the centre thereof and folded at the cut-out portion to form a pair of open-ended tubes joined at one end by a portion of the tubular material adjacent the cut-out, the cut-out portion facing outwardly from the joined end of the pair of tubes.

The tubular material may, for example, be translucent vinyl tubing of surgical non-toxic quality.

Suture threads are preferably provided enabling tape to be applied to the nose so as to hold the device safely and securely in position.

The device can be readily cut to size, smeared with medication if required, and inserted. It may be supplied in a sterile package ready for immediate use.

An embodiment of the invention will now be described by way of example with reference to the accompanying drawings in which:

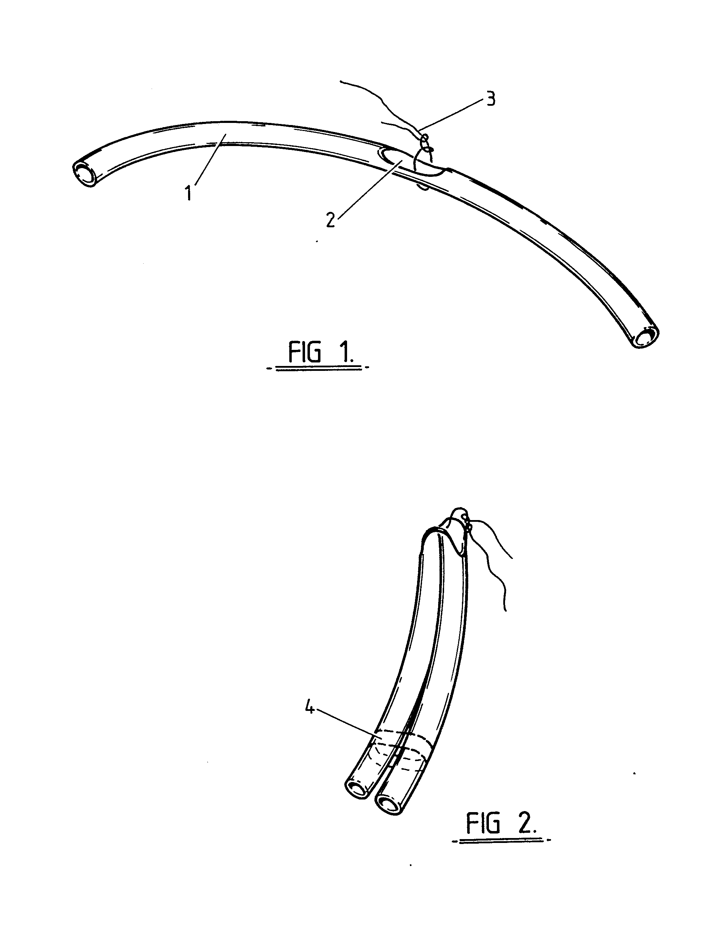

Figure 1 is a side view of a preferred form of the device of the invention;

Figure 2 is a view of the device of Figure 1 in use.
The device shown in the drawings comprises vinyl tubing
(1) conveniently about 180 mm long, and with a bore of about
6mm and an external diameter of about 7.6mm.  An oval slit
or slot (2) measuring in length say 18 mm, width of
6mm, at the centre of the oval is made in the tubing
midway between the open ends.  A surgical suture (3) is
threaded through the sector remaining after the slot is made,
tied and has two loose threads each about 40mm in length.
The tubing is shaped so as to approximate to a segment of
a circle in such a way that, on bringing the two open ends
together, the oval slot opens up to form two separate airways
with the threads in the exterior position.
In use the two open ends are inserted with forceps into
each nostril, the arc of the tubing facing away from the
operator and the inside of the arc facing the patient.
After insertion, the threads are automatically in an
exterior position and are taped to the bridge of the nose
to hold the device securely and safely in position.
For packaging, the open ends of the tubing may be held
in position by a tie (4) with directions for cutting to size.
The unit may be packaged in suitable see-through film,
sealed and sterilised.
The device which is used to maintain nasal airway after
nasal surgery in the critical 24 hour post-operative period,
exerts gentle compression to the base of the septum preventing
post-operative haematoma.  It also assists in preventing respiratory arrest due to nasal obstruction. (Partial or complete nasal obstruction can result in excess carbon dioxide generation and depletion ofoxygen increasing the risk of post-operative cardiac arrest).

The device may also be used in surgical and medical applications in which obstruction of the nasal passages occurs or may occur.

One advantage of the device of this invention is the ease with which it can be inserted, with or without lubrication. Medical lubrication of a therapeutic nature may be used.

It can easily and painlessly be fixed in position and because it is light in weight adds to patient comfort. The length of the device can be easily adjusted by simply cutting to meet individual patients needs. Further ease of removal is simple and painless, yielding not tissue trauma or bleeding.

Another advantage is that the device may be supplied, ready for use in a sterile package. The unit is not re-usuable and disposal is simple.

## CLAIMS:

1. A device for maintaining airways in nasal passages comprising a length of tubular material having cut-out portion and being folded above said cut-out portion to form a pair of open-ended tubes joined at one end by the portion of the tubular material adjacent the cut-out, said cut-out portion facing outwardly from the joined end of the pair of tubes.

2. A device according to claim 1 provided with suture threads.

3. A device for maintaining airways in nasal passages, constructed and arranged substantially as hereinbefore described and with reference to and as illustrated in the accompanying drawings.

0153132

FIG 1.

FIG 2.

| | **DOCUMENTS CONSIDERED TO BE RELEVANT** | | EP 85300917.3 |

| Category | Citation of document with indication. where appropriate, of relevant passages | Relevant to claim | CLASSIFICATION OF THE APPLICATION (Int. Cl.4) |
|---|---|---|---|
| A | US - A - 3 643 660 (HUDSON)<br>* Abstract; fig. 1,2,3 *<br>-- | 1 | A 61 M 15/08 |
| A | US - A - 3 682 171 (DALI)<br>* Abstract; fig. 1,2,3 *<br>---- | 1 | |

|  | TECHNICAL FIELDS SEARCHED (Int. Cl.4) |
|---|---|
| | A 61 M<br>A 61 F |

| The present search report has been drawn up for all claims | | |
|---|---|---|
| Place of search<br>VIENNA | Date of completion of the search<br>18-04-1985 | Examiner<br>MIHATSEK |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503. 03.82